# EUROPEAN PATENT APPLICATION

(11) **EP 0 947 494 A1**
(43) Date of publication of application: **06.10.1999**
(21) Application number: 99105721.7
(22) Date of filing: 20.03.1999
(51) Int. Cl.: C07C 49/794, C07D 257/04, A61K 31/12, A61K 31/41

(54) **Derivatives of phenoxy acetic acid and phenoxymethyltetrazole having antitumor activity**

(30) Priority: 30.03.1998 EP 98105726
(71) Applicant: F. HOFFMANN-LA ROCHE AG, 4070 Basel (CH)
(72) Inventor: Di Domenico, Roberto, I-20127 Milano (IT); Hansen, Silke, 46145 Oberhausen (DE); Kaluza, Brigitte, 83670 Bad Heilbrunn (DE); Menta, Ernesto, I-20063 Cernuso sul Naviglio (IT); Schumacher, Ralf, 82377 Penzberg (DE)
(74) Representative: Mezger, Wolfgang, Dr.

(57) **Abstract**

The present intention relates to the use derivatives of phenoxy acetic acid and of phenoxymethyl tetrazole of formula (I) 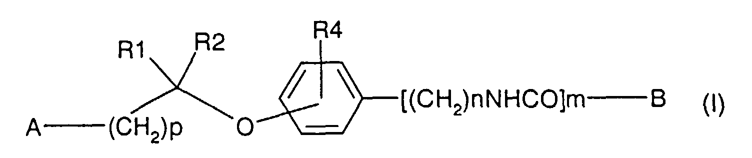 wherein:
- the -O-C(R1)(R2)-(CH₂)ₚ-A group can be in ortho, meta or para position;
- A is selected from -COOH, -COO-(C₁-C₄)alkyl, -CN or a group of formula 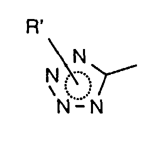 in which R' is hydrogen or (C₁-C₄)alkyl;
   or the group A-(CH₂)ₚ-C(R1)(R2)- is selected from phenyl, benzyl or (indolyl)methyl, which may be subsituted by R4 groups;
- p is 0, 1 or 2;
- R1 and R2 are independently selected from hydrogen or (C₁-C₈)alkyl or they form, together with the carbon atom to which they are linked, a (C₃-C₇)cycloalkyl group;
- R4 are from 0 to 2 substituents independently selected from chlorine, bromine, iodine, fluorine, linear or branched (C₁-C₈)alkyl, hydroxy, (C₁-C₄)alkoxy, (C₁-C₄)acyl groups; or the group 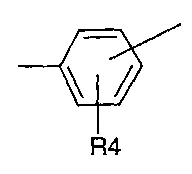 in formula (I) is a naphtyl group which may be on its turn substituted by R4 groups;
- n is an integer from 1 to 4;
- m is 0 or 1;
- B is selected from linear or branched C₁-C₁₀ alkyl, -CO-C(R3)=CH-R, -CH=C(R3)-CO-Ar, -CO-CH(R3)-CH₂-R or
- CO-CH(R3)-CH₂-NR5R6 when m is 0 or is -CH=C(R3)-CO-Ar when m is 1;
- R is selected from hydrogen, -Ar or -CO-Ar;
- R3 is hydrogen or a (C₁-C₈)alkyl group;
- R5 and R6 are independently a (C₁-C₄)alkyl group or they form, together with the nitrogen atom to which they are linked, a piperidino, piperazino, (C₁-C₈)alkylpiperazino, morpholino or thiomorpholino group;
- Ar is a phenyl group which can be unsubstituted or substituted with from 1 to 3 groups independently selected from chlorine, bromine, iodine, fluorine, linear or branched (C₁-C₈)alkyl, hydroxy, (C₁-C₄)alkoxy, (C₁-C₄)acyl groups, stereoisomers thereof or salts thereof with pharmaceuticaly acceptable acids or basis, for the preparation of a medicament having MDM2 antagonistic activity,
new compounds of the above formula, and pharmaceutical compositions containing at least one of those compounds.

## Description

The present invention relates to derivatives of phenoxy acetic acid and of phenoxymethyl tetrazole having anti-tumor properties, which act as antagonists of MDM2 activity. In particular, these compounds act as antagonists of the interaction of MDM2 with p53, thereby leading to an intracellular increase of active p53, thus allowing p53 protein to promote apoptosis in cancer cells.

The proto-oncoprotein MDM2 (mouse double minute 2, the human equivalent protein sometimes is designated as HDM2) is aberrantly expressed in a number of human tumors (Oliner et al. 1992, Nature 358, 80-83; Leach et al. 1993, Cancer Res. 54, 794-799; Ebert et al. 1994, Int. J. Oncol. 5, 1279-1284; Bueso-Ramos et al. 1993, Blood 82, 2617-2623; Chilosi et al. 1994, Blood 84, 4295-4300; Marchetti et al. 1995, Diagn. Mol. Pathol. 4, 93-97; McCann et al. 1995, Brit. J. Cancer 71, 981-985; Cordon-Cardo et al. 1994, Cancer Res. 54, 794-799). MDM2 forms a negative autoregulatory loop with p53 by binding to its N-terminal activation domain (Kussie et al. 1996, Science 274, 948-953), thereby inhibiting the functions of p53 (Momand et al. 1992, Cell 69, 1237-1245; Oliner et al. 1993, Nature 362, 857-860; Barak et al. 1992, EMBO J. 11, 2115-2121; Finlay 1993, Mol. Cell. Biol. 13, 301-306; Chen et al. 1996, Mol. Cell. Biol. 16, 2445-2452; Haupt et al. 1996, EMBO J. 15, 1596-1606) and promoting the proteolytic degradation of p53 (Kubbutat et al. 1997, Nature 387, 299-302; Haupt et al. 1997, Nature 387, 296-299; Midgley et al. 1997, Oncogene 15, 1179-1189). Interference with this autoregulatory loop between MDM2 and p53 therefore can be used to increase the concentration of active p53 within mammalian cells. Tumor cells in many instances are depleted in active p53, which leads to defective cell cycle and apoptosis regulation. However, only a fraction of tumor cells carries mutational defects in p53. In the remaining fraction of tumor cells with wild type p53, the equilibrium concentration of active p53 therefore can be increased by interfering with the interaction of MDM2 and p53. Peptidic model antagonists of the MDM2-p53 interaction have been used to show the restoration of p53 activity both in vitro (Böttger et al. 1997, J. Mol. Biol. 269, 744-756) and in mammalian cells (Böttger et al. 1997, Current Biology 7, 860-869). In addition, inhibition of MDM2 expression with synthetic nucleic acids has been shown to lead to increased levels of active p53 and thereby to increased sensitivity of cell lines to cytostatic drugs (Chen et al. 1998, Proc. Natl. Acad. Sci USA 95, 195-200). Lack of functional p53 is the most common molecular defect correlating with resistance of tumors to chemo- or radiotherapy. MDM2 disregulation has been implicated as a cause in this phenomenon (Kondo et al. 1995, Oncogene 10, 2001-2005; Blaydes et al. 1997, Oncogene 14, 1859-1868). Agents increasing the intracellular concentration of active p53 in tumor cells by interfering with the MDM2 p53 interaction therefore have therapeutic utility in sensitizing tumor cells for chemo- or radiotherapy. In tumor types particularly sensitive to increases in functional p53 (Hansen et al. 1995, Oncogene 11, 2535-2545), agents of this type will be sufficient to induce apoptosis on their own.

In addition to effects mediated via increase of intracellular concentrations of active p53, mdm2 antagonists are able to exert effects on the cell cycle regulation of mammalian cells, which are independent of p53. Transcription from E2F-dependent promoters is activated by mdm2, which interacts with E2F at the same binding site and with a homologous epitope as with p53 (Piette et al. 1997, Oncogene 15, 1001-1010; Brown et al. 1993, Mol. Cell. Biol. 13, 6849-6857). Therefore, mdm2 promotes general proliferation by enhancing S-phase passage (Leveillard and Wasylyk 1997, J. Biol. Chem. 272, 30651-30661), enhances expression of angiogenic mitogens (Kondo et al. 1996, Oncogene 13, 1773-1779) and inhibits differentiation (Fiddler et al. 1996, Mol. Cell. Biol. 16, 5048-5057). Inhibitors of mdm2 will therefore be therapeutically useful in mdm2 disregulated tumors independent of their p53 status.

It has now been found, that O-substituted phenol derivatives which carry an acidic moiety linked via an alkylene chain to the phenol oxygen atom are effective antagonists of MDM2 activity, in particular antagonists of the MDM2 p53 interaction.

Chalcone derivatives with a carboxymethoxy substituent on one of the two phenyl rings have been reported in literature to have antiulcer activity (Pol. J. Chem., vol. 65(2-3), 369-75, 1991; DE 3537207, Biorex; Chem. Pharm. Bull., vol. 27(12), 2943-53, 1979; FR 2383157, Biorex; JP 54019948, Taisho), bactericidal activity (Pharmazie, vol. 44(3), 190-1, 1989; Hacettepe Univ. Eczacilik Fak. Derg., vol. 11(1), 1-11, 1991), hypolipemic activity (FR 2639043; US 3,994,955, Searle; T'ai-wan Yao Hsueh Tsa Chih, vol. 27(1-2), 12-16, 1975), diuretic activity (Eur. J. Med. Chem.-Chim. Ther., vol. 16(6), 551-5 and 556-62, 1981, and BE 639727), pesticidal activity (CA 904291, Dow Chem.), or to lower fibrinogen level in the blood (DE 4327365, Boehringer Mannheim).. No antitumor activity has been reported so far.

The chalcone derivatives with antitumor activity reported in the state of the art do not carry a carboxymethoxy or tetrazolylmethoxy moiety on the phenyl ring and are known to act as antitubulinic agents (US 4,904,697, Merrell Dow).

Object of the present invention is the use of compounds of formula (I): 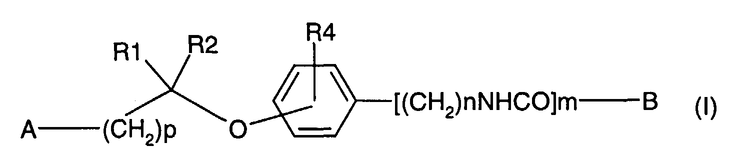 wherein:
- the -O-C(R1)(R2)-(CH₂)ₚ-A group can be in ortho, meta or para position;
- A is selected from -COOH, -COO-(C₁-C₄)alkyl, -CN or a group of formula 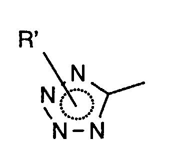 in which R' is hydrogen or (C₁-C₄)alkyl;
   or the group A-(CH₂)ₚ-C(R1)(R2)- is selected from phenyl, benzyl or (indolyl)methyl, which may be subsituted by R4 groups;
- p is 0, 1 or 2;
- R1 and R2 are independently selected from hydrogen or (C₁-C₈)alkyl or they form, together with the carbon atom to which they are linked, a (C₃-C₇)cycloalkyl group;
- R4 are from 0 to 2 substituents independently selected from chlorine, bromine, iodine, fluorine, linear or branched (C₁-C₈)alkyl, hydroxy, (C₁-C₄)alkoxy, (C₁-C₄)acyl groups; or the group 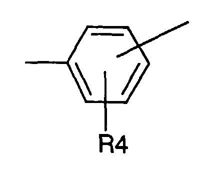 in formula (I) is a naphthyl group which may be on its turn substituted by R4 groups;
- n is an integer from 1 to 4;
- m is 0 or 1;
- B is selected from linear or branched C₁-C₁₀ alkyl, -CO-C(R3)=CH-R, -CH=C(R3)-CO-Ar, -CO-CH(R3)-CH₂-R or
- CO-CH(R3)-CH₂-NR5R6 when m is 0, or is -CH=C(R3)-CO-Ar when m is 1;
- R is selected from hydrogen, -Ar or -CO-Ar;
- R3 is hydrogen or a (C₁-C₈)alkyl group;
- R5 and R6 are independently a (C₁-C₄)alkyl group or they form, together with the nitrogen atom to which they are linked, a piperidino, piperazino, (C₁-C₈)alkylpiperazino, morpholino or thiomorpholino group;
- Ar is a phenyl group which can be unsubstituted or substituted with from 1 to 3 groups independently selected from chlorine, bromine, iodine, fluorine, linear or branched (C₁-C₈)alkyl, hydroxy, (C₁-C₄)alkoxy, (C₁-C₄)acyl groups, stereoisomers thereof or salts thereof with pharmaceutically acceptable acids or basis, for the preparation of a medicament having MDM2 antagonistic activity, preferably for the treatment of mdm2 disregulated tumors, for instance sarcomas.

Another object of the present invention are new compounds of formula (I) as above defined, with the proviso that, when m is 0, A is selected from -COO-(C₁-C₄)alkyl, -CN or a group of formula 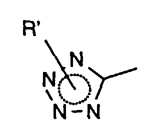 in which R' is hydrogen or (C₁-C₄)alkyl; and when m is 0 and A is a -COO-(C₁-C₄)alkyl group, B can only be a group of formula -CO-CH(R3)-CH₂-NR5R6.

Preferred compounds of formula (I) are those in which the -O-C(R1)(R2)-(CH₂)ₚ-A group is in para position.

Particularly preferred compounds of formula (I) are those in which R4 is from 1 to 2 chlorine atoms or those in which Ar is a phenyl substituted with from 1 to 2 chlorine atoms.

Even more particularly preferred compounds of formula (I) are those in which m is 0, R is hydrogen and A is a tetrazole group.

The most preferred compounds of formula (I) are:

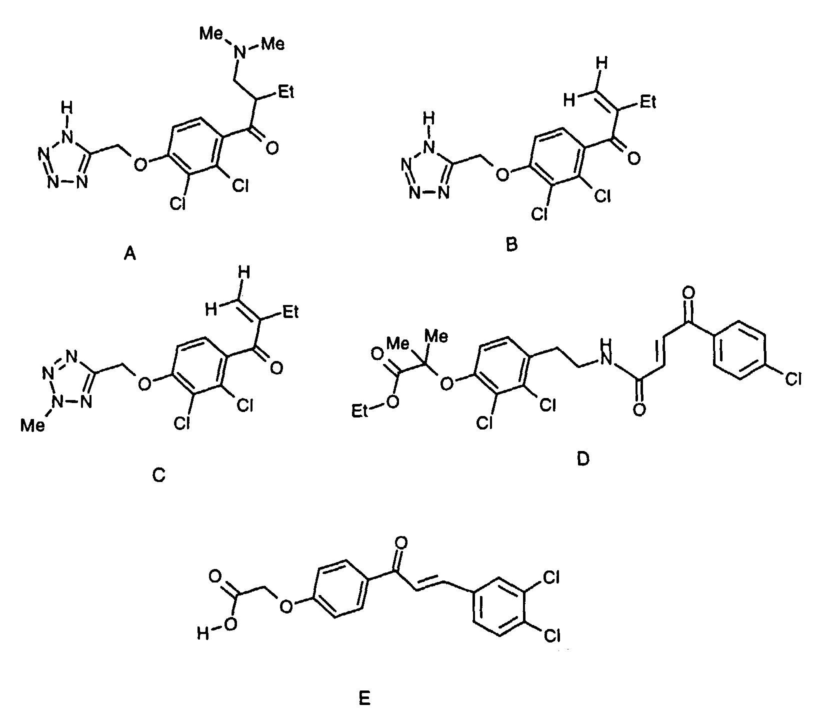

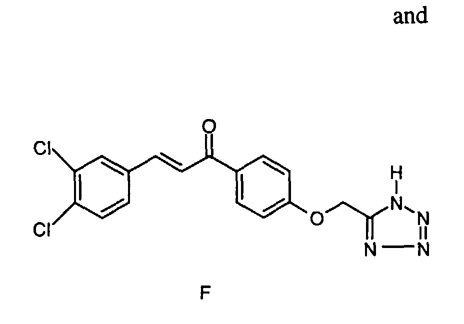

### PREPARATION OF THE COMPOUNDS OF THE INVENTION

The compounds of formula (I) in which m is 0 and B is a linear or branched C₁-C₁₀ alkyl, -COCH₃, -CO-C(R3)=CH-R or a -CH=C(R3)-CO-Ar group can be prepared starting from the intermediates of formula (II): 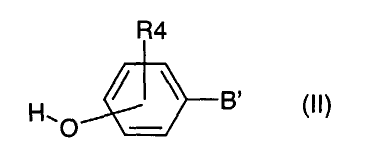 wherein R4 has the above meanings and B' is a linear or branched C₁-C₁₀ alkyl, -COCH₃, -CO-C(R3)=CH-R or a -CH=C(R3)-CO-Ar group, by reaction with an acid ester of formula (III):

Hal-C(R1)(R2)-(CH₂)ₚ-COO-(C₁-C₄)alkyl (III)

or with a nitrile of formula (III'): 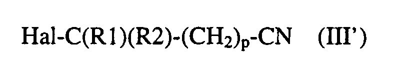 wherein R1, R2 and p have the above meanings and Hal is a chlorine, bromine or iodine atom, or with a group of formula (III''): 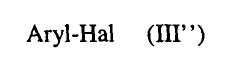 wherein Hal is a chlorine, bromine or iodine atom, and aryl is selected from benzyl, (indolyl)methyl and phenyl activated by a group which can be easily removed after the reaction, such as a chromium(0)-tricarbonyl group.

The reaction of intermediates of formula (II) with intermediates of formula (III) or (III') can be performed in a solvent, preferably an aprotic dipolar solvent such as dimethylformamide or dimethylsulfoxide, and in the presence of a base such as a carbonate of an alkaline or alkaline-earth metal. The reaction temperature preferably ranges from room temperature to 100°C.

The product of the reaction of intermediates of formula (II) wherin B' is not -COCH₃ with intermediates of formula (III) is already a compound of formula (I) and can be converted in another compound of formula (I) with A = -COOH by hydrolysis of the ester group. Such a hydrolysis reaction may be preferably performed in the presence of a base, such as an alkaline or alkaline-earth carbonate or hydroxide in a solvent such as an alcohol.

The product of the reaction of intermediates (II) wherein B' is not -COCH₃ with intermediates (III') is converted into a compound of formula (I) in which A is a tetrazole group as depicted above, by reaction with sodium azide in a solvent such as dimethylformamide, and optionally by alkylating the nitrogen atom in 1 or 2 position of the tetrazole ring by means of a suitable alkylating agent, such dimethylsulfate in the presence of a base.

Alternatively, the compounds of formula (I) where A is a tetrazole group as above defined, m is 0 and B is -COC(R3)=CH-R where R3 is H, may be obtained in a three-step process, comprising a) reaction of intermediates (II) above, where B' is -COCH₃, with intermediates of formula (III'); b) reaction of the compound obtained in step a) with sodium azide in a solvent such as dimethylformamide, and optionally by alkylating the nitrogen atom in 1 or 2 position of the tetrazole ring by means of a suitable alkylating agent, such dimethylsulfate in the presence of a base; and c) reaction of the compound obtained in step b) with an aromatic aldehyde R-CHO in an inert solvent and in the presence of a base such an hydroxide of an alkaline or alkaline-earth metal and at temperatures ranging from room temperature to reflux.

Alternatively, the compounds of formula (I) in which A is a tetrazole group, m is 0 and B is -CO-C(R3)=CH₂, i.e. the compounds in which R is hydrogen, may be obtained from the compounds of formula (II'): 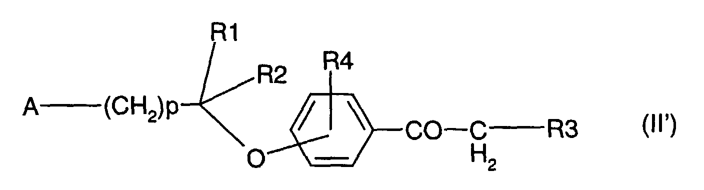 wherein A is a tetrazole group as depicted above and R1, R2, R3, R4 and p have the above meanings, by reaction with paraformaldheyde. By reaction of Formula II' with paraformaldehyde and a hydrochloride salt of an amine of formula HNR5R6, in which R5 and R6 have the above meanings, in the conditions of the Mannich reaction, and successive treatment with a weak base such as an alkaline or alkaline-earth hydrogencarbonate the compounds of formula (I) in which
B' = -CO-CH(R3)-CH₂-NR5R6 as hydrochloride salts are obtained.

The compounds of formula (II) in which B' is a group of formula -COC(R3)=CH-R with R = Ar or R = -CO-Ar can be obtained from compounds of formula (IV): 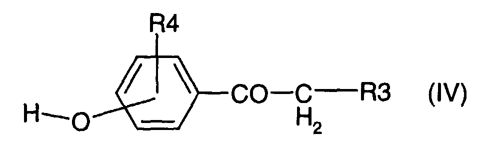 by reaction with an aldehyde of formula Ar-CHO or Ar-CO-CHO in an inert solvent and in the presence of a base such as an hydroxide of an alkaline or alkaline-earth metal and at temperatures ranging from room temperature to reflux.

Analogously, the compounds of formula (II) in which B' is a group of formula - CH=C(R3)-CO-Ar can be obtained from compounds of formula Ar-CO-CH₂-R3 with an aldehyde of formula (IV'):

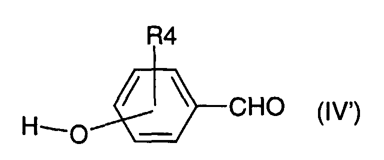

The compounds of formula (IV), (IV'), II with B' C₁-C₁₀ alkyl, Ar-CHO, Ar-CO-CHO and Ar-CO-CH₂-R3 are known compounds which can be prepared according to methods well known to the skilled chemist, or are even commercial products.

The compounds of formula (II') wherein A is a tetrazole group can be prepared from the compounds of formula (IV) by reaction with compounds of formula (III') and successively with sodium azide as shown above.

The compounds of formula (I) in which m = 0 and B is a -CO-CH(R3)-CH₂-R group can be prepared from the compounds of formula (II) in which B' is -CO-C(R3)=CHR by catalytic hydrogenation of the double bond and subsequent reaction with the intermediates of formula (III) or (III') as shown above. . The compound of formula (I) obtained by reaction with intermediate (III') may optionally be converted into another compound of formula I in which A is a tetrazolyl group by reaction with sodium azide, as shown above.

The compounds of formula (I) in which m is 1 and B is -CH=C(R3)CO-Ar can be obtained from the compounds of formula (V): 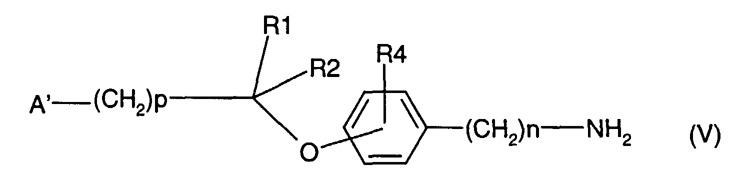 in which p, n, R1, R2 and R4 have the above meanings and A' has the meanings of A with exclusion of -COOH, by reaction with an intermediate of formula (VI):

Ar-CO-C(R3)=CH-COOH (VI)

Compounds wherein B is -CO-C(R3)=CH-R or -CO-CH(R3)-CH₂-R can be prepared in an analogous way. Such a reaction is performed by activating the carboxylic group of compounds (VI), for example via a mixed anhydride or a acyl chloride, or in the presence of a suitable condensing agent such as dicyclohexyl carbodiimide. The compounds so obtained are converted into other compounds of formula (I) by hydrolysis in basic conditions of the ester group, when A' = -COO-(C₁-C₄)alkyl, or by reaction with an azide, when A' = -CN, following the procedures depicted before.

The compounds of formula (V) can be prepared by esterification in acidic conditions of the corresponding acids (in which A' is -COOH), that are on their turn prepared according to the method described in Synthesis, (1997), 778-782, which is herein incorporated by reference. Suitable esterification conditions may be methanol in the presence of sulfuric acid in amount sufficient to salify the amino group and to catalyze the esterification reaction. By subsequent treatment with a weak base the free amino group can be restored.

The compounds of formula (VI) can be prepared according to the method described in Am. Soc., 70, 3359 (1948), which is herein incorporated by reference.

### BIOLOGICAL ACTIVITY OF THE COMPOUNDS OF THE INVENTION

The compounds of this invention interact with MDM2 protein, in particular human MDM2 protein, and inhibit the interaction of MDM2 with other proteins, in particular the interaction of MDM2 with p53. MDM2 has a variety of functions, the major one being to control p53 activity during cell cycle (reviewed by Piette et al. 1997, Oncogene 15, 1001-1010). MDM2 proteins form a hydrophobic pocket in their amino-terminal domain, which accommodates a peptidic epitope present on the amino-terminus of p53 (Kussie et al. 1996, Science 274, 948-953). This interaction between the N-terminus of p53 and the N-terminal domain of MDM2 is the key prerequisite for MDM2 to exert its control over p53 activity. Compounds binding to the hydrophobic pocket of the N-terminal domain of MDM2 therefore act as antagonists of the MDM2 mediated p53 inhibition and degradation. By this mechanism the levels of active p53 can be increased, which renders in particular tumor cells susceptible to p53 mediated induction of apoptosis and cell cycle arrest. Up to now only peptides and proteins have been available to demonstrate the feasibility of this mode of intervention (Böttger et al. 1997, J. Mol. Biol. 269, 744-756; Böttger et al. 1997, Current Biology 7, 860-869). The compounds of this invention now provide for the first time low molecular chemical entities able to interrupt the MDM2-p53 interaction.

In addition, the compounds of this invention are able to inhibit mdm2 from interacting with its N-terminal domain with other proteins having homologous interaction sites, such as E2F-1. The compounds of this invention are therefore able to exert antiproliferative or sensitizing effects on tumor cells, independent of the p53 status of the tumor cell (Example 15).

Furthermore, the compounds of this invention are particularly specific for interacting with MDM2. Within a mammalian cell, there exist several proteins with hydrophobic pockets able to accommodate compounds or hydrophobic residues. One example of such proteins is glutathione S-transferase (GST) (Reinemer et al. 1992, J. Mol. Biol. 227, 214-226; Cameron et al. 1995, Structure 3, 717-727; McTigue et al. 1995, J. Mol. Biol. 246, 21-27). It has been observed within this invention, that certain compounds are able to interact both with MDM2 and GST. An example of such a compound is ethacrynic acid, which has been described previously as an inhibitor of GST, binding to a hydrophobic pocket of this protein (Oakley et al. 1997, Biochemistry 36, 576-585; Ploemen et al. 1993, Xenobiotica 23, 913-923). Within this invention, an interaction of ethacrynic acid with MDM2 was surprisingly observed. This invention therefore provides assays to analyze the differential binding activity of compounds to MDM2 and GST, respectively. This invention further provides the technology to identify compounds with high binding affinity for mdm2 and low or preferably absent binding affinity for GST. Compounds with high inhibitory activity of the MDM2-p53 interaction and comparatively low or absent GST inhibitory activity are particularly preferred compounds for induction of a therapeutic anti-tumor effect based on inhibition of mdm2, because many tumors give rise during a cycle of chemotherapy to resistant tumor cell populations, which in many instances have upregulated the enzyme GST (Chen and Waxman 1994, Biochem. Pharmacol. 47, 1079-1087; Pickett and Lu 1989, Annu.Rev.Biochem. 58, 743-764). Interaction of a compound both with mdm2 and GST thereby will lead to a depletion of compound available for mdm2 inhibition due to competition. A compound of this type is for instance LSM 83177 (compound of Example 12, also called compound E), which has been found within this invention to be a potent sensitizing agent for tumor cells independent of their GST status (Example 15). The chemical structure of LSM 83177 is:

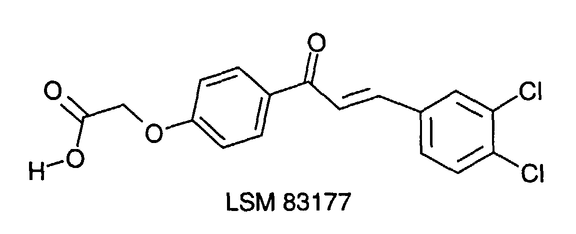

In addition, low or absent GST inhibitory activity is a desired property of a therapeutically useful mdm2 antagonist, because toxic side effects such as diuresis, hyperglycemia and hypercalcemia can be associated with inhibition of GST (O'Dwyer et al. 1991, Cancer Res. 51, 6059-6065; Oakley et al. 1997, Biochemistry 36, 576-585).

The following Examples 12-15 illustrate how the biological activity of the compounds of the present invention may be determined.

The compounds of the present invention can be administered in doses ranging from 0.01 mg to 0.4 g per kilogram of body weight daily. A preferred dosage regimen to obtain best results is that which provides for the use from about 1 mg to about 50 mg per kilogram of body weight daily, employing unitary doses so that to administer in 24 hours from about 70 mg to about 3.5 g of the active compound to a patient having approximately 70 kg of body weight. Such a dosage regimen may be adjusted to achieve the best therapeutical effect. For example, doses may be administered taking into account the therapeutical situation of the patient. The active compound may be administered by oral, intravenous, intramuscular or subcutaneous route.

The pharmaceutical compositions of the present invention contain therapeutical effective amounts of at least one compound of the invention in admixture with pharmaceutically compatible excipients.

Oral compositions will generally include an inert diluent or an edible carrier. They can be included in gelatin capsules or compressed into tablets. Other oral administration forms are capsules, pills, elixirs, suspensions or syrups.

The tablets, pills, capsules and similar compositions can contain the following ingredients (in addition to the active compound): a binder such as microcrystalline cellulose, tragacanth or gelatin; an excipient such as starch or lactose; a disintegrating agent such as alginic acid, primogel, maize starch and the like; a lubricant such as magnesium stearate; a fluidifier such as colloidal silicon dioxide; a sweetening agent such as sucrose or saccharine or a flavoring agent such as mint flavor, methyl salicylate or orange flavor. When the composition selected is in form of capsules, it can contain in addition a liquid carrier such as a fat oil. Other compositions can contain various material which change the physical form thereof, for example coating agents (for tablets and pills) such as sugar or shellac. The material used in the preparation of the compositions should be pharmaceutically pure and non toxic at the used dosages.

For the preparation of pharmaceutical compositions for the parenteral administration, the active ingredient can be included in solutions or suspensions, which can comprise in addition the following components: a sterile diluent such as water for injections, saline solution, oils, polyethylene glycols, glycerin, propylene glycol or other synthetic solvents; antibacterial agents such as benzyl alcohol; antioxidants such as ascorbic acid or sodium bisulfite; chelating agents such as ethylenediaminotetracetic acid; buffers such as acetates, citrates or phosphates and agents for adjusting the tonicity of the solution, such as sodium chloride or dextrose. The parenteral preparation can be included in ampoules, mono-dose syringes, glass or plastic vials.

A further object of the present invention is to provide pharmaceutical compositions containing at least one compound of the invention in admixture with pharmaceutically suitable excipients.

### CHEMICAL EXPERIMENTAL PART

The invention is further illustrated by the following Preparations and Examples.

The numbering of the positions on the chalcone rings is as follows:

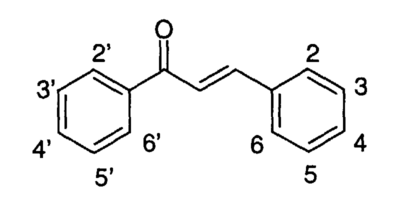

### Preparation 1: 4'-hydroxy-3-chlorochalcone

A solution of 1.36 g of 4-hydroxyacetophenone in 14 ml of ethanol is added at room temperature with 0.64 g of lithium hydroxide monohydrate, then further 6 ml of ethanol are added to facilitate the stirring. 1.17 ml of 3-chlorobenzaldheyde are added, then the reaction mixture is refluxed for 7 hours. The solvent is evaporated off under vacuum and the residue is dissolved in 15 ml of water. The solution is cooled at 0°C and added with 15 ml of 1 N hydrochloric acid. A yellow solid separates, which is allowed to stir for 1 hour, then it is filtered and dried under vacuum overnight. The solid is crystallized from 2.5 ml 96% ethanol, to give 0.41 g of the product, m.p. 163-165°C.
¹H-NMR in d6-DMSO: 6.90 ppm (d, 2H); 7.4 ppm (m, H); 7.6 ppm (d, 1H); 7.8 ppm (m, 1H); 8 ppm (d, 1H); 8.05 ppm (m, 1H); 8.1 ppm (d, 2H); 10.45 ppm (s, 1H).

### Preparation 2: 3',4'-dichloro-4-hydroxychalcone

A solution of 4-hydroxybenzaldheyde (1.22 g) in 20 ml of ethanol is added at room temperature with 0.63 g of lithium hydroxide monohydrate and 1.89 g of 3,4-dichloroacetophenone, then it is refluxed for 6 hours (after 4 hours a deep red solid separates) and at room temperature overnight. The solid is filtered off and the mother liquors are concentrated to dryness, then the residue is dissolved in a mixture of ethyl acetate and 1 N hydrochloric acid. The organic phase is separated, washed with brine, dried over sodium sulfate and concentrated to dryness. The residue (1.4 g) is redissolved in 30 ml of water, ethyl acetate and 1 N hydrochloric acid until acidic pH. After 30 minutes under stirring the organic phase is separated, washed twice with brine, dried over sodium sulfate and concentrated to dryness. The residue is crystallized from ethyl acetate (30 ml) under reflux, to give 0.946 g of the product as a yellow solid, m.p. 198-199°C.
¹H-NMR in d6-DMSO: 6.85 ppm (d, 2H); 7.7-7.9 ppm (m, 5H); 8.1 ppm (dd, 1H); 8.4 ppm (d, 1H); 10.15 ppm (s, 1H).

### Preparation 3: 3,4-dichloro-4'-hydroxychalcone

A solution of 4-hydroxyacetophenone (5.45 g) in 60 ml of ethanol is added with 3.36 g of lithium hydroxide monohydrate and 7 g of 3,4-dichlorobenzaldheyde, then it is refluxed for 2 hours. Further 3 g of 3,4-dichlorobenzaldheyde are added and the reaction mixture is refluxed for additional 2 hours and at room temperature overnight. The solid which separates is recovered by diltration; and redissolved into 50 ml of water and 50 ml of 1 N hydrochloric acid. A yellow solid separates, which is allowed to stir for 1 hour, then it is collected by filtration and dried under vacuum at 40°C for several hours, to give 7.3 g of the product, which is crystallized from a mixture of ethyl acetate (90 ml) and isopropanol (5 ml). 1.3 g of the product are obtained. Further 2.4 g are recovered by purification by silica gel chromatography of the mother liquors concentrated to dryness, m.p. 190-192°C.
¹H-NMR in d6-DMSO: 6.9 ppm (d, 2H); 7.65 ppm (d, 1H); 7.7 ppm (d, 1H); 7.8 ppm (m, 1H); 8.05 ppm (d, 1H); 8.1 ppm (d, 2H); 8.3 ppm (s, 1H); 10.4 ppm (s, 1H).

### Preparation 4: 3,4-dichloro-4'-hydroxy-dihydrochalcone

A solution of 0.6 g of 3,4-dichloro-4'-hydroxychalcone in 10 ml of ethanol and 3 ml of dioxane is added with 0.14 g of 10% palladium on charcoal, then it is hydrogenated for 1 hour 15 minutes. The catalyst is filtered off through a celite plug and the reaction mixture is concentrated to dryness. The residue gives after crystallization from diethyl ether 0.124 g of the product, m.p. 128-130°C. Further 0.221 g of the product are obtained by purification by silica gel chromatography (eluant petroleum ether/ethyl acetate 8 : 2) of the mother liquors concentrated to dryness.
¹H-NMR in d6-DMSO: 2.9 ppm (t, 2H); 3.3 ppm (t, 2H); 6.8 ppm (d, 2H); 7.25 ppm (d, 1H); 7.5-7.6 ppm (m, 2H); 7.9 ppm (d, 2H); 10.3 ppm (s, 1H).

### Preparation 5: 2,3-dichloro-4-butyroylphenol

144 g of 2,3-dichloroanisole are dissolved in 288 ml of carbon disulfide and added with 92.3 g of butyroyl chloride. Under stirring and cooling with ice, 115 g of aluminum trichloride are added portionwise, keeping the temperature below 25°C. The reaction mixture is allowed to stand at room temperature for 1 hour, then it is heated at 45°C for 45 minutes. After adding 280 ml of n-heptane and further 115 g of aluminum trichloride, the reaction mixture is allowed to react overnight, then the carbon disulfide is distilled off and further 200 ml of n-heptane are dropped. A solid separates which is heated under stirring at 80-90°C for 3 hours and at room temperature overnight. The solid is collected by filtration, then it is treated with 86 ml of concentrated hydrochloric acid and 1 l of water. The mixture is extracted three times with diethyl ether and the organic extracts are pooled and washed with water and with 750 ml of 5% sodium hydroxide. The extracts are then treated with concentrated hydrochloric acid and the oil which separates is allowed to crystallize under cooling. 114.7 g of the product are obtained.

### Preparation 6: (2,3-dichloro-4-butyroylphenoxy)acetonitrile

45 g of 2,3-dichloro-4-butyroylphenol are mixed with 26.7 g of potassium carbonate and 16 g of chloroacetonitrile in 190 ml of dimethylsulphoxide and the mixture is heated under stirring at 85°C for 2 hours 30 minutes. The reaction mixture is then quenched with 490 g of ice. An oil separates which is extracted four times with diethyl ether, then the organic phase is treated with 400 ml of 5% sodium hydroxide and washed with water. The organic phase is then dried over magnesium sulfate and concentrated to dryness, to give 45.2 of an oil which, after distillation at 188°C and 0.8 mmHg, gives 40 g of the product.

### Preparation 7: 5-[((2,3-dichloro-4-butyroyl)phenoxy)methyl]tetrazole

40 g of (2,3-dichloro-4-butyroylphenoxy)acetonitrile are mixed with 11.5 g of sodium azide and 9.5 g of ammonium chloride in 294 ml of dimethylformamide. The reaction mixture is heated under stirring at 120-130°C for 30 minutes, then the solvent is evaporated under reduced pressure (at 80°C). The residue is treated with 1.3 l of water under stirring. An oil separates which then crystallizes and it is collected by filtration. 44 g of the rough product are obtained, which are crystallized from a mixture of 400 ml of methanol and 200 ml of water. 34.8 g of the product are obtained, m.p. 135-137°C.
Elem. Anal. (% calcd/found): C 45.73/45.43; H 3.84/3.73; N 17.78/17.32; Cl 22.50/22.56.

### Preparation 8: 5-[((2,3-dichloro-4-butyroyl)phenoxy)methyl]-1-methyltetrazole and 5-[((2.3-dichloro-4-butyroyl)phenoxy)methyl]-2-methyltetrazole

8.53 g of 5-[((2,3-dichloro-4-butyroyl)phenoxy)methyl]tetrazole are dissolved with 120 ml of water and 480 ml of acetone. 31.8 g of sodium carbonate are added, then 52.8 g of dimethyl sulfate are added dropwise and the reaction mixture is allowed to react for 20 hours. The mixture is then poured into water and the organic solvent is evaporated under reduced pressure at 90°C. From the aqueous phase an oil separates which crystallizes upon cooling. The solid is filtered and dried to give 7.93 g of a 1 : 1 mixture of the title tetrazoles. The solid is recrystallized from a mixture 1 : 1 of benzene/cyclohexane, to give 2.55 g of 5-[((2,3-dichloro-4-butyroyl)phenoxy)methyl]-1-methyltetrazole. The filtrate is concentrated to dryness and the residue is crystallized from cyclohexane, to give 3.5 g of 5-[((2,3-dichloro-4-butyroyl)phenoxy)methyl]-2-methyltetrazole.

### Preparation 9: 4-(cyanomethoxy)acetophenone

To a solution of 4-hydroxyacetophenone (0.272 g) in DMF (8 ml, dried over molecular sieves), 2-chloroacetonitrile (0.164 ml) followed by potassium carbonate (0.636 g) were added and the obtained mixture was heated at 60°C for 1 hr.

After cooling to room temperature and dilution with water (40 ml), the solution was brought to pH 4 with HCl and then extracted with ethyl acetate. The organic layer was washed once with brine, dried over Na₂SO₄ and evaporated to dryness, to give 4-(cyanomethoxy)acetophenone as a dark solid (0.354 g). This material was used as such for the next step.
¹H-NMR (CDCl₃): 2.58 ppm (s, 3H); 4.85 ppm (s, 2H), 7.05 ppm (m, 2H); 8.0 ppm (m, 2H).

### Preparation 10: 4-(5-tetrazolylmethoxy)acetophenone

Sodium azide (0.165 g) and ammonium chloride (0.107 g) were added to a solution of 4-(cyanomethoxy)acetophenone (0.349 g) in DMF (5 ml; dried over molecular sieves) and the obtained mixture was heated at 70°C for 2.5 hours.

The mixture was cooled to 0°C and treated with 1N HCl (50 ml). After stirring at 0°C for 0.5 h the yellowish precipitate was recovered by filtration and dried in vacuum at 40°C overnight, to give 4-(5-tetrazolylmethoxy)acetophenone (0.32 g) as a brownish solid. This material was used as such for the next step.
¹H-NMR (DMSO-D₆): 2.45 ppm (s, overlapping with DMSO signal); 5.6 ppm (s, 2H); 7.15 ppm (d, 2H); 8.0 ppm (d, 2H); 16.9 ppm (br. s, 1H).

### Example 1: 4'-(ethoxycarbonylmethoxy)-3-chlorochalcone

A solution of 4'-hydroxy-3-chlorochalcone (1.3 g) in 24 ml of anhydrous dimethylformamide is added at room temperature and under nitrogen atmosphere with 1.73 g of potassium carbonate and 0.84 ml of ethyl bromoacetate. The reaction mixture is heated at 60°C for 1 hour 30 minutes, then it is diluted with 100 ml of water and extracted with ethyl acetate (3x20 ml). The organic extracts are pooled and washed with brine, then the organic phase is dried over sodium sulfate and concentrated to dryness to give a residue (1.83 g) which is purified by silica gel chromatography (eluant petroleum ether/ethyl acetate 7.5:2.5), obtaining 0.66 g of the product, m.p. 68-70°C.
¹H-NMR in CDCl₃: 1.3 ppm (t, 3H); 4.3 ppm (q, 2H); 4.75 ppm (s, 2H); 7.0 ppm (d, 2H); 7.34 ppm (m, 2H); 7.5 ppm (m, 1H); 7.55 ppm (d, 1H); 7.6 ppm (m, 1H); 7.7 ppm (d, 1H); 8.0 ppm (d, 2H).

### Example 2: 4'-(carboxymethoxy)-3-chlorochalcone

A suspension of 0.345 g of 4'-(ethoxycarbonylmethoxy)-3-chlorochalcone in 4 ml of ethanol and 4 ml of water is added at room temperature with 0.212 g of sodium carbonate and kept at room temperature overnight. Further 1.5 ml of ethanol and 1.5 ml of water are added and the reaction mixture is refluxed for 1 hour, then it is cooled to room temperature while a yellowish solid separates. The solid is recovered by filtration, redissolved in water/ethyl acetate and added with 1 N hydrochloric acid until acidic reaction. The organic phase is separated, washed with brine, dried over sodium sulfate and concentrated to dryness, to give 0.29 g of a yellowish solid, which crystallized from ethyl acetate (20 ml) to give 0.12 g of the product, m.p. 180-181°C.
¹H-NMR in d6-DMSO: 4.9 ppm (s, 2H); 7.05 ppm (d, 2H); 7.5 ppm (m, 2H); 7.7 ppm (d, 1H); 7.85 ppm (m, 1H); 8.05 ppm (d, 1H); 8.1 ppm (s, 1H); 8.2 ppm (d, 2H); 13.1 ppm (s, 1H).

### Example 3: 3',4'-dichloro-4-(ethoxycarbonylmethoxy)chalcone

A solution of 3',4'-dichloro-4-hydroxychalcone (0.586 g) in 9 ml of anhydrous dimethylformamide is added, at room temperature and under nitrogen atmosphere, with 0.69 g of potassium carbonate and with 0.334 g of ethyl bromoacetate. The reaction mixture is heated at 60°C for 3 hours, then it is cooled to room temperature and poured into a mixture of 40 ml of water and 20 ml of ethyl acetate. The mixture is kept under stirring until all the solid dissolves, then the organic phase is separated, washed with ethyl acetate, dried over sodium sulfate and concentrated to dryness, to give 0.737 of the product as a yellow solid.
¹H-NMR in d6-DMSO: 1.2 ppm (t, 3H); 4.2 ppm (q, 2H); 4.9 ppm (s, 2H); 7.0 ppm (d, 2H); 7.7-8.0 ppm (m, 5H); 8.15 ppm (dd, 1H); 8.4 ppm (d, 1H).

### Example 4: 3',4'-dichloro-4-(carboxymethoxy)chalcone

A suspension of 0.38 g of 3',4'-dichloro-4-(ethoxycarbonylmethoxy)chalcone in 4 ml of ethanol and 4 ml of water is added with 0.212 g of sodium carbonate and it is refluxed for 3 hours 30 minutes. The reaction mixture is allowed to cool to room temperature and the solid which separates is collected by filtration , then it is partitioned between water, added with 1 N hydrochloric acid until acidic pH, and ethyl acetate. The organic phase is separated, washed with brine, dried over sodium sulfate and concentrated to dryness. The residue is crystallized from ethyl acetate (10 ml) to give 0.158 g of the product, m.p. 203-206°C.
¹H-NMR in d6-DMSO: 4.8 ppm (s, 2H); 7.0 ppm (d, 2H); 7.7-8.0 ppm (m, 5H); 8.1 ppm (dd, 1H); 8.4 ppm (d, 1H); 13.1 ppm (s, 1H).

### Example 5: 3,4-dichloro-4'-(ethoxycarbonylmethoxy)dihydrochalcone

A solution of 3,4-dichloro-4'-hydroxychalcone (0.221 g) in 3.5 ml of anhydrous dimethylformamide is added with 0.125 ml of ethyl bromoacetate and with 0.26 g of potassium carbonate, then it is heated at 60°C for 1 hour 45 minutes. The reaction mixture is allowed to cool to room temperature and it is diluted with 20 ml of water and 20 ml of ethyl acetate. The mixture is acidified with 1 N hydrochloric acid to pH 3-4, then the organic phase is separated, washed with brine, dried over sodium sulfate and concentrated to dryness. 0.268 g of the product is obtained as yellowish oil.
¹H-NMR in CDCl₃: 1.3 ppm (t, 3H); 3.0 ppm (t, 2H); 3.2 ppm (t, 2H); 4.25 ppm (q, 2H); 4.6 ppm (s, 2H); 6.9 ppm (d, 2H); 7.05 ppm (dd, 1H); 7.3 ppm (m, 2H); 7.9 ppm (d, 2H).

### Example 6 - 3,4-dichloro-4'-(carboxymethoxy)-dihydrochalcone

A solution of 0.268 g of 3,4-dichloro-4'-(ethoxycarbonylmethoxy)dihydrochalcone in 3 ml of ethanol is added with 3 ml of water, then with 0.15 g of sodium carbonate and it is heated at 70°C for 2 hours. The reaction mixture is concentrated to dryness, then it is added with 4 ml of water and 2 ml of 1 N hydrochloric acid, until pH 2-3. After 30 minutes under stirring, the solid is recovered by filtration, washed with water on the filter and dried under vacuum at 50°C overnight. 0.196 g of the product is obtained as a white powder, m.p. 148-150°C.
¹H-NMR in d6-DMSO: 2.9 ppm (t, 2H); 3.3 ppm (t, 2H); 4.7 ppm (s, 2H); 7.0 ppm (d, 2H); 7.25 ppm (dd, 1H); 7.6 ppm (m, 2H); 7.9 ppm (d, 2H); 13.1 ppm (s, 1H).

### Example 7 - 5-[((2,3-dichloro-4-(2'-methylenebutyroyl))phenoxy)methyl]tetrazole

39.2 g of 5-[((2,3-dichloro-4-butyroyl)phenoxy)methyl]tetrazole, 4.33 g of paraformaldheyde and 11.2 g of dimethylamine hydrochloride in 1 ml of acetic acid are heated at 80-90°C for 2 hours. After cooling to room temperature, the reaction mixture is partitioned between water and diethyl ether. The aqueous solution is treated with sodium hydrogencarbonate and the solid which separates is collected by filtration. The solid (22 g) are treated with 220 ml of water and 220 ml of 2 N sodium hydroxide and the mixture is heated until complete dissolution and for one additional hour. The aqueous phase is acidified and extracted with diethyl ether. The organic extracts are pooled, dried over magnesium sulfate and concentrated to dryness. The residue (7.66 g) is crystallized from 70 ml of benzene. 5.3 g of the product are obtained.
Elem. Anal. (% calcd/found): C 47.72/47.01; H 3.70/3.52; N 17.13/16.78; Cl 21.67/21.43.

### Example 8: 5-[((2,3-dichloro-4-(2'-methylenebutyroyl))phenoxy)methyl]-1-methyltetrazole

9.5 g of 5-[((2,3-dichloro-4-butyroyl)phenoxy)methyl]-1-methyltetrazole, 1.01 g of paraformaldheyde and 2.53 g of dimethylamine hydrochloride in 5 drops of acetic acid are heated at 80-90°C for 2 hours. The mixture is concentrated to dryness and it is poured into 100 ml of water. 200 ml of a sodium hydrogencarbonate solution are added and the mixture is kept under stirring for 4 hours until a solid separates. The solid is filtered to give 6.3 g of a rough material which is recrystallized from 130 ml of a 1 : 1 mixture benzene/cyclohexane. 5.58 g of the product are obtained, m.p. 124-125°C.
Elem. Anal. (% calcd/found): C 49.28/49.69; H 4.14/4.33; N 16.42/16.41; Cl 20.79/20.53.

### Example 9: 5-[((2,3-dichloro-4-(2'-methylenebutyroyl))phenoxy)methyl]-2-methyltetrazole

8 g of 5-[((2,3-dichloro-4-butyroyl)phenoxy)methyl]-2-methyltetrazole, 0.89 g of paraformaldheyde and 2.29 g of dimethylamine hydrochloride in 4 drops of acetic acids are heated at 80-90°C for 2 hours. The mixture is then concentrated to dryness and diluted with water. A solid separates which is collected by filtration. The filtrate is treated with a sodium hydrogencarbonate solution and heated with a water bath. The solid which forms is collected by filtration, to give 7.76 g of residue which is crystallized from 400 ml of cyclohexane. 4.16 g of the product are obtained.
Elem. Anal. (% calcd/found): C 49.28/49.11; H 4.14/4.55; N 16.42/16.13; Cl 20.79/20.54.

### Example 10: Ethyl 2-(4-(2-(3-(4-chlorobenzoyl)acryloylamino)ethyl)phenoxy)-2-methylpropionate (compound D)

A solution of 10.3 g of β-(4-chlorobenzoyl)acrylic acid are dissolved in 100 ml of tetrahydrofuran, added with 7.02 ml of triethylamine and cooled to -15°C. 5.25 ml of ethyl chloroformate are added dropwise and the reaction mixture is kept under stirring for 15 minutes. A solution of 12.6 g of ethyl 2-(4-(2-aminoethyl)phenoxy)-2-methylpropionate in 20 ml of tetrahydrofuran is dropped into the reaction mixture, which is then kept for 30 minutes at - 15°C, for 2 hours 30 minutes at 0°C and at room temperature overnight. The mixture is concentrated to dryness and redissolved into diethyl ether. The organic phase is washed with 2 N hydrochloric acid, then with 2 N sodium hydroxide and finally with water, then it is dried over sodium sulfate and concentrated to dryness. The residue, after crystallization from a few diethyl ether, gives 10 g of the product, m.p. 76-77°C.
Elem. Anal. (%calcd/found): C 64.94/64.77; H 5.90/5.59; N 3.15/3.14; Cl 7.98/8.12.

### Example 11: 2-(4-(2-(3-(4-chlorobenzoyl)acryloylamino)ethyl)phenoxy)-2-methylpropionic acid

20 g of ethyl 2-(4-(2-(3-(4-chlorobenzoyl)acryloylamino)ethyl)phenoxy)-2-methylpropionate are dissolved in 70 ml of methanol and added with 100 ml of 1 N potassium hydroxide solution. The stirring is continued for 3 hours heating at 45°C. After cooling to room temperature, the mixture is concentrated to dryness, redissolved with diethyl ether and treated with 1 N hydrochloric acid. The organic phase is separated, dried over sodium sulfate and concentrated to dryness, to give 8 g of the product as a brown amorphous solid.

### Example 12: 3,4-dichloro-4'-(carboxymethoxy)chalcone (compound E or LSM 83177)

A solution of 3,4-dichloro-4'-hydroxychalcone (0,586 g) (preparation 3) in 9 ml of anhydrous dimethylformamide is added, at room temperature and under nitrogen atmosphere, with 0.69 g of potassium carbonate and with 0.334 g of ethyl bromoacetate. The reaction mixture is heated at 60 °C for 3 hours, then it is cooled to room temperature and poured into a mixture of 40 ml of water and 20 ml of ethyl acetate. The mixture is kept under stirring until all the solid dissolves, then the organic phase is separated, washed with ethyl acetate, dried over sodium sulfate and concentrated to dryness, to give 3,4-dichloro-4'-(ethoxycarbonylmethoxy)chalcone. 3,4-dichloro-4'-(ethoxycarbonylmethoxy)chalcone is saponified analogous to Example 4 to 3,4-dichloro-4'-(carboxymethoxy)chalcone.

### Example 12A: 3,4-dichloro-4'-(5-tetrazolylmethoxy)calchone (compound F)

Under a nitrogen atmosphere, lithium hydroxide hydrate (0.059 g) was added to a stirred suspension of 4-(4-tetrazolylmethoxy)acetophenone (0.15 g) in ethanol (2 ml), followed by the addition of 3,4-dichlorbenzaldehyde (0.123 g). The obtained mixture was heated to reflux for 2 hours. After cooling to room temperature, the precipitate was filtered and resuspended in water (2 ml). The suspension was brought to acid pH by treatment with 1N HCl and stirred for two hours. The solid was separated by filtration and recrystallised from MeOH, to give 3,4-dichloro-4'-(tetrazolylmethoxy)calchone as a yellow powder (117 mg).
m.p. > 230°C
¹H-NMR (DMSO-D₆): 5.45 ppm (s, 2H); 7.25 ppm (d, 2H); 7.65 ppm (d, 1H); 7.78 ppm (m, 1H); 7.88 ppm (m, 1H), 8.10 ppm (d, 1H), 8.20 ppm (d, 2H); 8.35 ppm (m, 1H).

### BIOLOGICAL EXPERIMENTAL PART

### Example 13

Preparation of MDM2: A DNA fragment encoding human MDM2 amino acids 1-118 was obtained by PCR and inserted into a modified plasmid vector (pQE40; QIAGEN Inc., Chatsworth CA, USA) under the control of a T5 promoter suitable for expression in E. coli in combination with the pUBS 520 repressor (Brinkmann et al., 1989, Gene, 85, 109-114). BL21 cells (E. coli) were grown in LB medium at 37°C and induced for expression by addition of 1mM IPTG, allowing >15 hours for accumulation of MDM2 protein. Cells are harvested, disrupted by French Press and insoluble MDM2 protein is prepared by standard protocols. MDM2 inclusion bodies are then solubilized and subsequently refolded in 100mM Tris, pH7; 1mM EDTA; 10mM DTT (according to Rudolph et al. 1997, Protein Function: A practical approach, 2nd ed., IRL Press, 57-99), typically yielding a MDM2 preparation of > 80% purity suitable for interaction analysis of compounds. Further purification is performed by standard chromatographic procedures (hydrophobic interaction chromatography). The longer MDM2 fragment 1 - 213aa was obtained and purified in analogy to the described protocol.

### Example 14

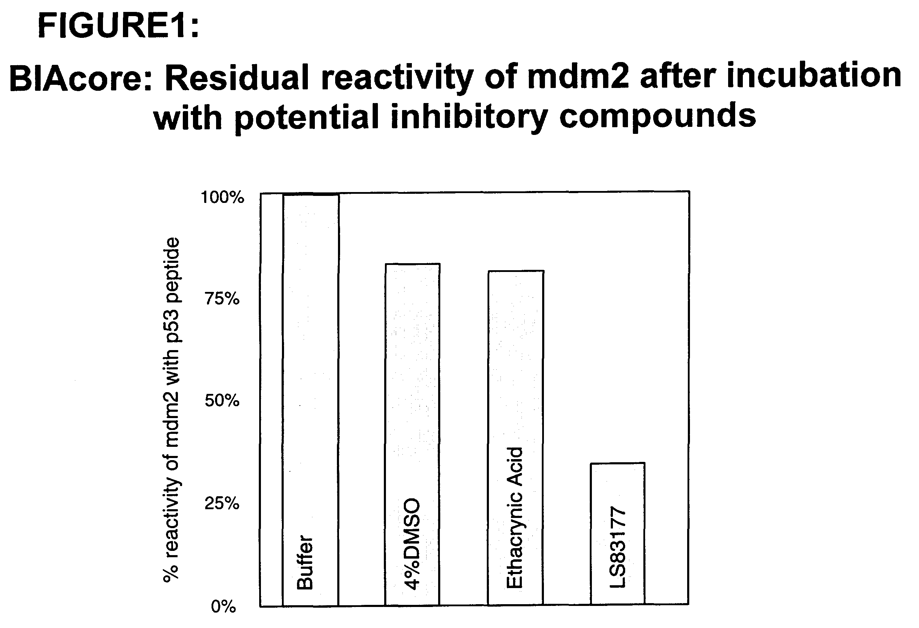

BIACORE analysis of the interaction between MDM2 and p53: To quantify the effect of selected compounds on the p53 binding properties of MDM2 protein biosensor measurements using BIACORE 2000 are performed. BIACORE 2000 is a biosensor system delivered from BIACORE AB. The technology of this biosensor is based on the optical phenomenon of surface plasmon resonance (SRP), which detects changes in refractive index of the solution close to the surface of the sensor chip. The refractive index is directly correlated to the mass concentration in the layer and increases when analyte binds to a surface immobilized ligand. Experiments are performed under continuous flow conditions. The SPR response expressed in resonance units (RU) is recorded continuously versus time resulting in a sensorgram. When one interaction is completed, the surface can be regenerated using solutions which remove bound analyte without affecting the activity of the bound ligand. A N-terminally biotinylated peptide (5µM in PBST buffer) corresponding to amino acids 19 to 32 of wild type human p53 obtained from Genosys Biotechnologies (Cambridge) was captured at a flow rate of 5µl/min for 6 minutes on a SA-sensor chip (sensor chip pre-immobilized with streptavidin, BIACORE AB). 40 µl of mdm2 1-213 (100nM in PBST) were mixed with 40 µl of the sample (40µM in PBST with 2 % DMSO). After 15 minutes incubation at 10°C, 30 µl of the mixture were injected on the sensor chip with a flow rate of 10µl/min. After additional 4 minutes rinsing the surface with PBST buffer, a signal was recorded. By comparing the signals of the samples with those of buffer, the inhibitory effects of the samples could be evaluated. The sensor surface was regenerated by rinsing with 100mM HCl and 100mM H₃PO₄. Figure 1 shows the inhibitory effects of selected compounds on the interaction of mdm2 with the p53 derived peptide as relative units.

### Example 15

Spectrophotometric GST activity analysis: Human cell lines such as human colon carcinoma cell line HT29 are grown as monolayers at 37°C, in 5% CO₂, in DMEM supplemented with antibiotics and 10% fetal calf serum and are passaged twice a week GST activity in the cytosol is determined according to Habig et al. (Habig, W.H., Pabst, M.J. & Jakoby, W.B., J. Biol. Chem. 249, 7130-7139, 1974) using 1-chloro-2,4-dinitrobenzene (CDNB) and Glutathione. GST catalyzes the conjugation of CDNB with glutathione and results in a CDNB-glutathione product with a strong molar absorption at 340 nm. The change of absorption is monitored for 5 min.

1 x 10⁶ cells are collected and washed once with ice-cold phosphate-buffered saline at 1000 rpm for 10 min at 4°C. Cell pellets are resuspended in 200 µl ice-cold phosphate-buffered saline, and are sonicated for 2 min on ice. The sonicate is then centrifuged at 11750 g, 4°C for 15 min in an Eppendorf Centrifuge, and the supernatant is assayed for GST activity. The inherent inhibitory effect of Ethacrynic Acid (EA) and selected MDM2 antagonists on the catalytic activity of HT29 cytosolic GST activity is examined by addition of the drugs directly to cell extracts immediately before addition of glutathione.

Table 1 shows the inhibitory effect of EA and LSM 83177 on GST activity in extracts from HT29 cells.

### Example 16

Biological assay for radiosensitizing activity of MDM2 antagonistic compounds: Human tumor cell lines containing wild-type p53 and low levels of GST, such as MCF7 (breast carcinoma) or mutant p53 and a high GST content, such as MCF7 ADR (adriamycin-resistant breast carcinoma) are cultivated in RPMI medium, supplemented with 10% fetal calf serum to a semi-confluent state.

In order to determine the radiosensitizing activity of selected compounds on these cells a minimal toxic dose was determined as 2Gy (gray: J/kg) with a ¹³⁷Cesium source at room temperature. Following irradiation of monolayer cells in exponential growth phase with 2 Gy, cells were incubated with various concentrations of the selected compounds for 2-6 h. Cells were trypsinized and seeded in appropriate dilutions into 6 well plates. Surviving cells were trypsinized after 12-13 days and cell number was determined by staining with trypane blue for living/dead cells. The protocol was established according to Khil et al., 1996, Int. J. Rad. Oncol. Biol. Phys. 34, 375-380. Table 2 shows the differential radiosensitizing activity obtained with selected compounds on MCF-7 cell lines with low and high GST content.

In MCF-7 cells a radiosensitization of both compounds, ethacrynic acid and LSM 83177, can be observed: the enhancement of irradiation by the drug is of a factor 1.5, when the drug is incubated for 2 hours onto the cells and of a factor 2, when incubated for 6 hours.

Radiosensitizing activity independent of the GST content of the target cell can be observed only with MDM2-specific compounds such as LSM 83177: Specifically targeting MDM2 leads to a radiosensitization in MCF-7 ADR cells due to MDM2-specific interactions with E2F and Rb, despite of the fact that mutant p53 cannot be activated in this cell line. Ethacrynic acid does not mediate any radiosensitization within MCF-7 ADR cells.

**Table 1**

| **Inhibition of GST activity in HT29 cells by potential inhibitory compounds** | | |
|---|---|---|
| Concentration | EA | LSM 83177 |
| [µg/ml] | [% GST activity] | |
| 20 | 0 | 0 |
| 10 | 4 | 26 |
| 8 | 17 | 46 |
| 5 | 36 | 73 |
| 2,5 | 48 | 95 |
| 2 | 57 | 100 |
| 1,3 | 64 | 100 |
| 1 | 84 | 100 |
| 0,4 | 91 | 100 |
| 0,2 | 99 | 100 |
| 0,04 | 100 | 100 |
| IC-50 [µg/ml] | 2,2 | 7,7 |

**Table 2**

| Compounds were incubated at a final concentration of 20 µg/ml with the respective cells. A- MCF-7 cells | | | | |
|---|---|---|---|---|
| | % living cells relative to control without drug treatment | | | |
| drug incubation time following irradiation | ethacrynic acid | 2 Gy + ethacrynic acid | LSM 83177 | 2 Gy + LSM 83177 |
| 0 hours | 100 | *100* | 100 | *100* |
| 2 hours | 52.3 | *42.7* | 64.1 | *41.8* |
| 6 hours | 43.1 | *20.5* | 36.4 | *17* |

| B- MCF-7ADR | | | | |
|---|---|---|---|---|
| | % living cells relative to control without drug treatment | | | |
| drug incubation time following irradiation | ethacrynic acid | 2Gy + ethacrynic acid | LSM 83177 | 2 Gy + LSM 83177 |
| 0 hours | 100 | *100* | 100 | *100* |
| 2 hours | 88.9 | *82.5* | 87.2 | *72.8* |
| 6 hours | 99 | *133.6* | 85.7 | *48.8* |

## Claims

1. Use of compounds of formula (I): 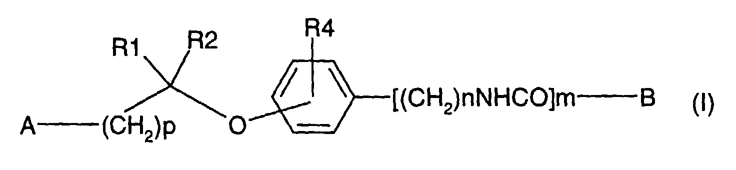 wherein:
- the O-C(R1)(R2)-(CH₂)ₚ-A group can be in ortho, meta or para position;
- A is selected from -COOH, -COO-(C₁-C₄)alkyl, -CN or a group of formula 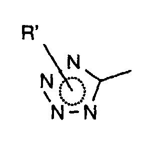 in which R' is hydrogen or (C₁-C₄)alkyl;
or the group A-(CH₂)ₚ-C(R1)(R2)- is selected from phenyl, benzyl or (indolyl)methyl, which may be substituted by R4 groups;
- p is 0, 1 or 2;
- R1 and R2 are independently selected from hydrogen or (C₁-C₈)alkyl or they form, together with the carbon atom to which they are linked, a (C₃-C₇)cycloalkyl group;
- R4 are from 0 to 2 substituents independently selected from chlorine, bromine, iodine, fluorine, linear or branched (C₁-C₈)alkyl, hydroxy, (C₁-C₄)alkoxy, (C₁-C₄)acyl groups; or the group 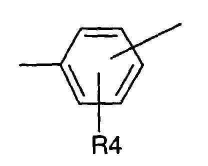 in formula (I) is a naphtyl group which may be on its turn substituted by R4 groups;
- n is an integer from 1 to 4;
- m is 0 or 1;
- B is selected from linear or branched C₁-C₁₀ alkyl, -CO-C(R3)=CH-R, -CH=C(R3)-CO-Ar, -CO-CH(R3)-CH₂-R or
- CO-CH(R3)-CH₂-NR5R6 when m is 0, or is -CH=C(R3)-CO-Ar when m is 1;
- R is selected from hydrogen, -Ar or -CO-Ar;
- R3 is hydrogen or a (C₁-C₈)alkyl group;
- R5 and R6 are independently a (C₁-C₄)alkyl group or they form, together with the nitrogen atom to which they are linked, a piperidino, piperazino, (C₁-C₈)alkylpiperazino, morpholino or thiomorpholino group;
- Ar is a phenyl group which can be unsubstituted or substituted with from 1 to 3 groups independently selected from chlorine, bromine, iodine, fluorine, linear or branched (C₁-C₈)alkyl, hydroxy, (C₁-C₄)alkoxy, (C₁-C₄)acyl groups, stereoisomers thereof or salts thereof with pharmaceutically acceptable acids or basis, for the preparation of a medicament having MDM2 antagonistic activity.

2. The use of claim 1, for the treatment of tumors.

3. The use of claim 2, in which the tumor to be treated is a sarcoma.

4. Compounds of formula (I): 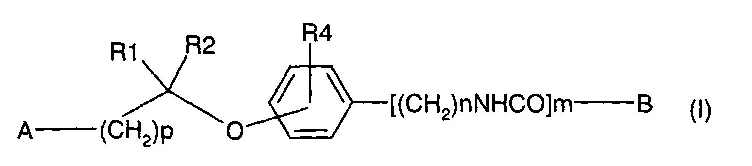 wherein:
- the -O-C(R1)(R2)-(CH₂)ₚ-A group can be in ortho, meta or para position;
- A is selected from -COOH, -COO-(C₁-C₄)alkyl, -CN or a group of formula 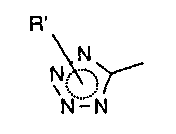 in which R' is hydrogen or (C₁-C₄)alkyl;
or the group A-(CH₂)ₚ-C(R1)(R2)- is selected from phenyl, benzyl or (indolyl)methyl, which may be substituted by R4 groups;
- p is 0, 1 or 2;
- R1 and R2 are independently selected from hydrogen or (C₁-C₈)alkyl or they form, together with the carbon atom to which they are linked, a (C₃-C₇)cycloalkyl group;
- R4 are from 0 to 2 substituents independently selected from chlorine, bromine, iodine, fluorine, linear or branched (C₁-C₈)alkyl, hydroxy, (C₁-C₄)alkoxy, (C₁-C₄)acyl groups; or the group 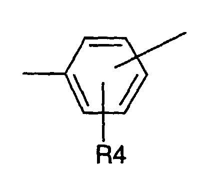 in formula (I) is a naphtyl group which may be on its turn substituted by R4 groups;
- n is an integer from 1 to 4;
- m is 0 or 1;
- B is selected from linear or branched C₁-C₁₀ alkyl, -CO-C(R3)=CH-R, -CH=C(R3)-CO-Ar, -CO-CH(R3)-CH₂-R or
- CO-CH(R3)-CH₂-NR5R6 when m is 0, or is -CH=C(R3)-CO-Ar when m is 1;
- R is selected from hydrogen, -Ar or -CO-Ar;
- R3 is hydrogen or a (C₁-C₈)alkyl group;
- R5 and R6 are independently a (C₁-C₄)alkyl group or they form, together with the nitrogen atom to which they are linked, a piperidino, piperazino, (C₁-C₈)alkylpiperazino, morpholino or thiomorpholino group;
- Ar is a phenyl group which can be unsubstituted or substituted with from 1 to 3 groups independently selected from chlorine, bromine, iodine, fluorine, linear or branched (C₁-C₈)alkyl, hydroxy, (C₁-C₄)alkoxy, (C₁-C₄)acyl groups,
stereoisomers thereof or salts thereof with pharmaceutically acceptable acids or basis,
with the proviso that, when m is 0, A is selected from -COO-(C₁-C₄)alkyl, -CN or a group of formula 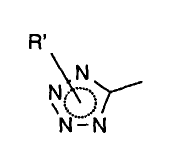 in which R' is hydrogen or (C₁-C₄)alkyl; and when m is 0 and A is a -COO-(C₁-C₄)alkyl group, B can only be a group of formula -CO-CH(R3)-CH₂-NR5R6.

5. Compounds according to claim 4, in which m is 0, R is hydrogen and A is a tetrazole group

6. Compounds according to claim 4 wherein Ar is a phenyl substituted with from 1 to 2 chlorine atoms.

7. Compounds according to any of claims 4 to 6, in which R4 is from 1 to 2 chlorine atoms

8. Compounds according to any of claim 4 to 7, in which the -O-C(R1)(R2)-(CH₂)ₚ-A group is in para position.

9. Compounds of one of the following formulae:

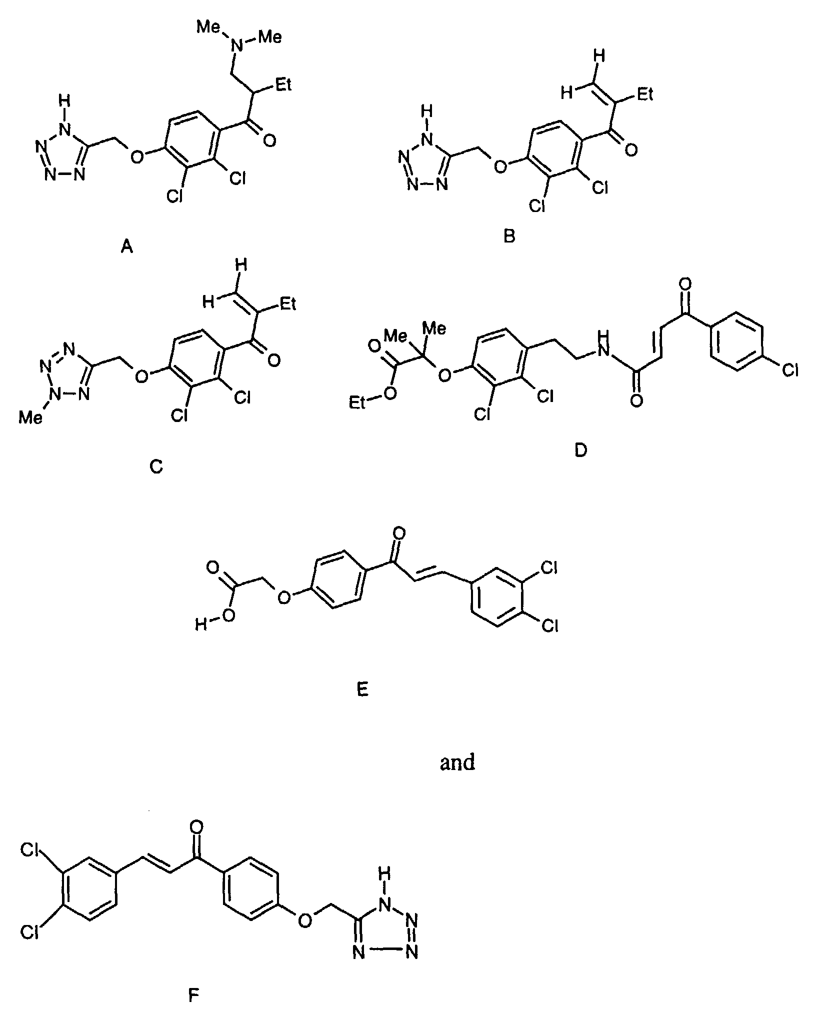

10. Pharmaceutical compositions containing at least one compound of any of claims 4 to 9 in admixture with pharmaceutically suitable excipients.
